# EUROPEAN PATENT APPLICATION

(11) **EP 4 282 426 A1**
(43) Date of publication of application: **29.11.2023**
(21) Application number: 22742212.8
(22) Date of filing: 20.01.2022
(51) Int. Cl.: A61K 38/21, A61K 45/06, A61P 31/14, A61P 31/20, A61P 31/22, A61P 35/00

(54) **METHOD AND PHARMACEUTICAL COMBINATION FOR PREVENTING CANCER RECURRENCE**

(30) Priority: 21.01.2021 CN 202110084507
(71) Applicant: Xiamen Amoytop Biotech Co., Ltd., Xiamen, Fujian 361028 (CN); Biosteed Gene Transformation Tech.Co., Ltd., Xiamen, Fujian 361028 (CN)
(72) Inventor: LIAO, Xiaojin, Xiamen, Fujian 361028 (CN); WU, Hanzhou, Xiamen, Fujian 361028 (CN); CHU, Hongran, Xiamen, Fujian 361028 (CN); ZHU, Peijuan, Xiamen, Fujian 361028 (CN); XIAO, Qingjiang, Xiamen, Fujian 361028 (CN); ZHANG, Tingting, Xiamen, Fujian 361028 (CN); YIN, Fenghong, Xiamen, Fujian 361028 (CN); WU, Linying, Xiamen, Fujian 361028 (CN); ZHUANG, Lu, Xiamen, Fujian 361028 (CN); ZHOU, Weidong, Xiamen, Fujian 361028 (CN); SUN, Li, Xiamen, Fujian 361028 (CN)
(74) Representative: Vossius & Partner Patentanwälte Rechtsanwälte mbB
(86) International application number: PCT/CN2022/072921
(87) International publication number: WO 2022/156733

(57) **Abstract**

The present invention relates to the field of biomedicine. Disclosed herein is a method for preventing cancer recurrence, comprising i) intermittently administering an interferon-based therapeutic agent, and optionally ii) administering an additional anticancer agent, to a subject. The present invention also relates to a pharmaceutical combination for use in said method.

## Description

### Technical Field

The present invention relates to the field of biomedicine. Disclosed herein is a method for preventing cancer recurrence, comprising i) intermittently administering an interferon-based therapeutic agent, and optionally ii) administering an additional anticancer agent, to a subject. The present invention also relates to a pharmaceutical combination for use in said method.

### Background

Interferons (IFN) are active proteins with multiple functions, and cytokines produced by monocytes and lymphocytes. With various biological activities, such as broad-spectrum anti-viral activity, influence on cell growth, differentiation, and regulation of immune functions, they are at present the most important anti-viral infection and anti-tumor biological products.

At present, interferons are categorized into Type I, Type II, and Type III interferons. The Type I interferons include IFN-α, IFN-β, and the like. The IFN-α interferons are mainly generated by monocytes-macrophages. In addition, B cells and fibroblasts may also synthesize the IFN-α interferons. The IFN-β interferons are mainly generated by fibroblasts. Both IFN-α and IFN-β interferons bind to the same receptors which are widely distributed in for example monocyte-macrophages, polymorphonuclear leukocytes, B cells, T cells, platelets, epithelial cells, endothelial cells and tumor cells. It is known that there are more than 23 subtypes of IFN α. There is only one subtype of IFN β. The Type II interferons, or γ interferons, are mainly generated by activated T cells (including Th0, Th1 cells and almost all CD8+ T cells) and NK cells, and belong to the so-called lymphokines. The IFN-γ may exist in the form of linking to an extracellular matrix, so it may control the cell growth by bystander effect. They may be distributed on the surface of almost all cells except mature red blood cells. There is only one subtype of IFN-γ. The Type III interferons mainly refer to the interferon λ.

Interferons, interferon mutants and interferon derivatives have been approved to be widely used in various treatments. Interferons and mutants that have been approved for human clinical treatment include interferon α 2a, interferon α 2b, interferon α 1b, compound interferon (Infergen), and interferon mutants (such as Novaferon), Interferon β, Interferon γ (1b), and the like. Interferon derivatives include Peginterferon α 2a, Peginterferon α 2b, integrated interferon and the like.

In addition, in recent years, with the discovery and elucidation of signal pathways such as TLRs, RLRs, and STING, a series of agonists that act on the above signal pathways to generate interferons have been discovered and elucidated, the use of which has also become a new direction for future interferon-based treatments. At present, this type of agonists has been used in the treatment of HBV and related tumors.

The Type I IFN is the first batch of immunotherapy drugs approved by FDA for clinical therapy of cancers. It provided the best curative effect for hematological system tumors, followed by for lymphatic system tumors, and the worst curative effect for solid tumors, such as malignant melanoma, ovarian tumor, colorectal cancer, etc., where the curative effect is no more than 20%. In addition, the combined application of interferon with chemotherapy, radiotherapy and immunotherapy is at present a common anti-tumor therapy with interferons. For example, combined application of interferon with 5-fluorouracil for treating liver cancer and colorectal cancer, combined application of interferon with cisplatin, carboplatin and the like for treating lung cancers. Although these studies have achieved some results, the overall effect is not so satisfactory, and the prognosis is poor.

In addition to anticancer treatment, an important way to reduce cancer mortality is to prevent cancer recurrence. However, there are no reports in the art on the successful application of interferon to prevent cancer recurrence.

### Summary of the Invention

In one aspect, the present invention provides a method for preventing cancer recurrence in a subject, comprising
i) administering an interferon-based therapeutic agent; and
ii) optionally, administering an additional anticancer agent,
to the subject.

In some embodiments, the method comprises administering to the subject at least 1 consecutive course of the interferon-based therapeutic agent.

In some embodiments, the method comprises intermittently administering to the subject a plurality of consecutive courses of the interferon-based therapeutic agent.

In some embodiments, the subject has been diagnosed as having cancer, but has undergone an anti-cancer treatment and is in remission. In some embodiments, the anticancer treatment is surgery, chemotherapy, radiation therapy, immunotherapy, or a combination thereof. In some embodiments, the remission is a complete remission, e.g., no visible lesion (tumor) is detected in the subject.

In some embodiments, the interferon-based therapeutic agent comprises an interferon or a mutant or derivative thereof, or comprises a nucleic acid molecule encoding an interferon or a mutant or a derivative thereof, or comprises a substance promoting the generation of an endogenous interferon.

In some embodiments, the interferon is a Type I, Type II or Type III interferon, such as interferon α, interferon β, interferon γ or interferon λ, preferably interferon α.

In some embodiments, the interferon-based therapeutic agent comprises interferon α 2a, interferon α 2b, interferon α 1b, interferon λ, or a mutant or derivative thereof.

In some embodiments, the interferon or the mutant or derivative thereof is PEGylated.

In some embodiments, the interferon-based therapeutic agent is selected from the group consisting of P1101, Pegberon, Pegasys, Pegintron, Infergen, Novaferon, INTRONA, Roferon-A, Hapgen, PEGINFER and Peginterferon λ.

In some embodiments, the interferon-based therapeutic agent comprises an agonist of the TLRs, RLRs, and STINGs signaling pathways.

In some embodiments, the interferon-based therapeutic agent is selected from the group consisting of GS-9620, GS-9688, RO7020531, RO6864018, TQ-A3334, JNJ-4964, SB9200, MIW815, DMXAA, MK-1454, and diABZI.

In some embodiments, in the consecutive treatment course, the interferon-based therapeutic agent is administered such that during substantially the entire course, the concentration of neopterin in the subject is higher than the concentration of neopterin before the first administration, for example approximately 110%, approximately 120%, approximately 130%, approximately 140%, approximately 150%, approximately 200%, approximately 250% or higher of the neopterin concentration before the first administration.

In some embodiments, the duration of the consecutive treatment course is the time period from the first administration to the last administration, plus about 5 in vivo half-lives of the therapeutic agent.

In some embodiments, the duration of the consecutive treatment course is from about 1 week to about 24 weeks, preferably from about 1 week to about 12 weeks, further preferably from about 1 week to about 8 weeks, and yet further preferably about 2 weeks to about 6 weeks.

In some embodiments, the interval between the consecutive treatment courses is from about 1 week to about 24 weeks, preferably from about 1 week to about 12 weeks, further preferably from about 1 week to about 8 weeks, and yet further preferably about 2 weeks to about 6 weeks.

In some embodiments, the duration of each of the consecutive treatment courses is about 1 week to about 8 weeks, and the interval between the consecutive treatment courses is about 1 week to about 8 weeks.

In some embodiments, the duration of each of the consecutive treatment courses is about 2 weeks to about 6 weeks, and the interval between the consecutive treatment courses is about 2 week to about 6 weeks.

In some embodiments, the interferon-based therapeutic agent is administered for 2-25 or more consecutive treatment courses.

In some embodiments, the durations of the plurality of consecutive treatment courses are substantially the same.

In some embodiments, the intervals between the consecutive treatment courses are substantially the same.

In some embodiments, the additional anticancer agent is preferably used for preventing cancer recurrence in the subject.

In some embodiments, the anticancer agent is
i) a chemotherapeutic agent, such as an alkylating agent an alkylating agent: Nimustine, Carmustine, Lomustine, Cyclophosphamide, Ifosfamide, glyciphosphoramide, semustine; an antimetabolite: deoxyfluoguanosine, doxifluguanidine, 5-fluorouracil, mercaptopurine, thioguanine, cytarabine, fluguanosine, tegafur, Gemcitabine, carmofur, hydroxyurea, methotrexate, UFT, Ancitabine, capecitabine; an anti-tumor antibiotic: actinomycin D, doxorubicin, daunorubicin, Epirubicin, mitomycin, pelomycin, pingyangmycin, pirarubicin; a chemotherapeutic anti-tumor animal and plant ingredient: irinotecan, harringtonine, hydroxycamptothecin, Vinorelbine, paclitaxel, albumin paclitaxel, taxotere, topotecan, vincristine, vindesine, Vindesine, vinblastine, teniposide, etoposide, elemene; such as anti-tumor drug hormones: Atamestane, Anastrozole, Aminoglutethimide, Letrozole, Formestane, Metasterone, Tamoxifen; a chemotherapeutic miscellaneous agent: asparaginase, carboplatin, cisplatin, Dacarbazine, Oxaliplatin, Loxadine, Eloxatin, Mitoxantrone, or Procarbazine; or
ii) an immune checkpoint inhibitor such as an inhibitor of PD-1, PD-L1, CTLA4, for example, an antibody selected from: Nivolumab, Pembrolizumab, Atezolizumab, Durvalumab, Avelumab; or
iii) a small molecule targeting drug, such as imatinib, gefitinib, bortezomib, erlotinib, sorafenib, lenalidomide, Sunitinib, dasatinib, nilotinib, lapatinib, pazopanib, everolimus, vandetanib, crizotinib, verofinib, ruxolitinib, axitinib, vismodegib, carfilzomib, regorafenib, bosutinib, tofacitinib, carbotinib, panatinib, pomalidomide, trametinib, dabrafenib, Afatinib, Icotinib, Ibrutinib, Ceritinib, Idelaris, Apatinib, Pabuccilib, Levatinib, Axitinib, Icotinib , Apatinib, sonidegib, cobimetinib, osimertinib, alectinib, ixazomib; or
iv) a tumor-associated antigen-specific antibody such as Rituxan, Herceptin;
preferably, the anticancer agent is selected from oxaliplatin, epirubicin, paclitaxel and gemcitabine, and more preferably is gemcitabine.

In some embodiments, the cancer is selected from leukemia (such as acute lymphocytic leukemia (ALL), acute myeloid leukemia (AML), chronic myeloid leukemia (CML), chronic lymphocytic leukemia, polycapillary leukemia), liver cancer, lung cancer, colorectal cancer, skin cancer, stomach cancer, breast cancer, prostate cancer, non-Hodgkin's lymphoma, melanoma, multiple myeloma, laryngeal papilloma, follicular lymphoma, AIDS-related Kaposi's sarcoma and renal cell carcinoma, preferably liver cancer, lung cancer, breast cancer, colorectal cancer or melanoma.

In one aspect, the present invention provides a pharmaceutical combination for use in preventing cancer recurrence in a subject, comprising an interferon-based therapeutic agent and an additional anticancer agent.

In some embodiments, the pharmaceutical combination is for use in preventing cancer recurrence in a subject by the method of the invention.

In some embodiments, the interferon-based therapeutic agent comprises an interferon or a mutant or derivative thereof, or comprises a nucleic acid molecule encoding an interferon or a mutant or a derivative thereof, or comprises a substance promoting the generation of an endogenous interferon.

In some embodiments, the interferon is a Type I, Type II or Type III interferon, such as interferon α, interferon β, interferon γ or interferon λ, preferably interferon α.

In some embodiments, the interferon-based therapeutic agent comprises interferon α 2a, interferon α 2b, interferon α 1b, interferon λ, or a mutant or derivative thereof.

In some embodiments, the interferon or the mutant or derivative thereof is PEGylated.

In some embodiments, the interferon-based therapeutic agent is selected from the group consisting of P1101, Pegberon, Pegasys, Pegintron, Infergen, Novaferon, INTRONA, Roferon-A, Hapgen, PEGINFER and Peginterferon λ.

In some embodiments, the interferon-based therapeutic agent comprises an agonist of the TLRs, RLRs, and STINGs signaling pathways.

In some embodiments, the interferon-based therapeutic agent is selected from the group consisting of GS-9620, GS-9688, RO7020531, RO6864018, TQ-A3334, JNJ-4964, SB9200, MIW815, DMXAA, MK-1454, and diABZI.

In some embodiments, the anticancer agent is
i) a chemotherapeutic agent, such as an alkylating agent an alkylating agent: Nimustine, Carmustine, Lomustine, Cyclophosphamide, Ifosfamide, glyciphosphoramide, semustine; an antimetabolite: deoxyfluoguanosine, doxifluguanidine, 5-fluorouracil, mercaptopurine, thioguanine, cytarabine, fluguanosine, tegafur, Gemcitabine, carmofur, hydroxyurea, methotrexate, UFT, Ancitabine, capecitabine; an anti-tumor antibiotic: actinomycin D, doxorubicin, daunorubicin, Epirubicin, mitomycin, pelomycin, pingyangmycin, pirarubicin; a chemotherapeutic anti-tumor animal and plant ingredient: irinotecan, harringtonine, hydroxycamptothecin, Vinorelbine, paclitaxel, albumin paclitaxel, taxotere, topotecan, vincristine, vindesine, Vindesine, vinblastine, teniposide, etoposide, elemene; such as anti-tumor drug hormones: Atamestane, Anastrozole, Aminoglutethimide, Letrozole, Formestane, Metasterone, Tamoxifen; a chemotherapeutic miscellaneous agent: asparaginase, carboplatin, cisplatin, Dacarbazine, Oxaliplatin, Loxadine, Eloxatin, Mitoxantrone, or Procarbazine; or
ii) an immune checkpoint inhibitor such as an inhibitor of PD-1, PD-L1, CTLA4, for example, an antibody selected from: Nivolumab, Pembrolizumab, Atezolizumab, Durvalumab, Avelumab; or
iii) a small molecule targeting drug, such as imatinib, gefitinib, bortezomib, erlotinib, sorafenib, lenalidomide, Sunitinib, dasatinib, nilotinib, lapatinib, pazopanib, everolimus, vandetanib, crizotinib, verofinib, ruxolitinib, axitinib, vismodegib, carfilzomib, regorafenib, bosutinib, tofacitinib, carbotinib, panatinib, pomalidomide, trametinib, dabrafenib, Afatinib, Icotinib, Ibrutinib, Ceritinib, Idelaris, Apatinib, Pabuccilib, Levatinib, Axitinib, Icotinib , Apatinib, sonidegib, cobimetinib, osimertinib, alectinib, ixazomib; or
iv) a tumor-associated antigen-specific antibody such as Rituxan, Herceptin;
preferably, the anticancer agent is selected from oxaliplatin, epirubicin, paclitaxel and gemcitabine, and more preferably is gemcitabine.

### Brief Description of the Drawings

FIG. 1 shows the SP Sepharose Fast Flow elution profile of mIFN-α4 fermentation supernatant.
FIG. 2 shows the non-reduced SDS-PAGE (14% separation gel, silver staining) electrophoresis results of SP Sepharose Fast Flow purified target mIFN-α4.
FIG. 3 shows the Q Sepharose Fast Flow purification elution profile of glycosyl-removed mIFN-α4.
FIG. 4 shows the non-reduced SDS-PAGE (14% separation gel, silver staining) electrophoresis results of Q Sepharose Fast Flow purified raw solution of glycosyl-removed mIFN-α4.
FIG. 5 shows the SP Sepharose Fast Flow purification elution profile of PEG-mIFN-α4.
FIG. 6 shows the non-reduced SDS-PAGE (silver staining) electrophoresis results of PEG-mIFN-α4 raw solution.
FIG. 7 shows the plasma concentration-time curve for a single subcutaneous injection of 1µg/mouse of PEG-mIFN-α4 to BALB/c mice.
FIG. 8. The tumor incidence curves of the comparative experiment of PEG-mIFN-α4 alone and PEG-mIFN-α4 in combination with gemcitabine on the prevention of recurrence of transplanted liver cancer H22 in mice.
FIG. 9. The survival curves of the comparison experiment of PEG-mIFN-α4 alone and PEG-mIFN-α4 in combination with gemcitabine for the prevention of recurrence of transplanted liver cancer H22 in mice.
FIG. 10. The tumor incidence curves of the comparison experiment of PEG-mIFN-α4 alone and PEG-mIFN-α4 in combination gemcitabine on the prevention of post-surgery recurrence of transplanted colorectal cancer CT26 in mice.
FIG. 11. Survival curves of the comparison experiment of PEG-mIFN-α4 alone and PEG-mIFN-α4 in combination gemcitabine on the prevention of post-surgery recurrence of transplanted colorectal cancer CT26 in mice.

### Detailed Description of the Invention

The inventors unexpectedly discovered that by administering interferon, for example, intermittently administering a plurality of courses of interferon, cancer recurrence can be prevented in patients at risk.

Therefore, in one aspect, the present invention provides a method for preventing cancer recurrence in a subject, comprising administering an interferon-based therapeutic agent to the subject.

In some embodiments, the method comprises administering to the subject at least 1 consecutive course of the interferon-based therapeutic agent. In some embodiments, the method comprises intermittently administering to the subject a plurality of consecutive courses of the interferon-based therapeutic agent.

As used herein, the "subject" is a mammal, such as a human.

In some embodiments, the subject has been diagnosed as having cancer, but has undergone an anti-cancer treatment and is in remission. In some embodiments, the anticancer treatment is surgery, chemotherapy, radiation therapy, immunotherapy, or a combination thereof. In some embodiments, the remission is a complete remission, e.g., no visible lesion (tumor) is detected in the subject.

As used herein, the "interferon" may be a human interferon, for example, a Type I, II or III interferon, such as interferon α, interferon β, interferon γ or interferon λ, preferably interferon α.

As used herein, the "interferon-based therapeutic agent" refers to a therapeutic agent capable of generating at least part of the effects of a natural interferon.

For example, the "interferon-based therapeutic agents" may include a nature isolated or a recombinantly generated interferon, such as a Type I interferon, preferably an interferon α. A suitable interferon α includes but is not limited to interferon α 2a, interferon α 2b or interferon α 1b.

The "interferon-based therapeutic agent" may also include an interferon mutant, such as Infergen (a recombinant integrated interferon).

The "interferon-based therapeutic agent" may also include an interferon derivative, such as PEGylated interferon or a mutant thereof, an albuminated interferon or a mutant thereof, and another protein and organic-modified interferon and a mutant thereof, and the like. Examples of the PEGylated modified interferon or the mutant thereof include, but are not limited to, peginterferon α 2a (e.g. Pegasys, 40kD bi-branched UPEG-NHS modified), peginterferon α 2b (e.g., Pegintron, 12kD linear PEG-SC modified), peginterferon α 2b (e.g., Pegberon, Y-type 40kD 2 Branched PEG modified), cultured interferon α-2 (e.g., PEGINFER), Peginterferon λ, P1101, and the like.

In some embodiments, the "interferon-based therapeutic agent" includes a long-acting interferon, such as a PEGylated interferon or a mutant thereof. In some embodiments, the "interferon-based therapeutic agent" includes a short-acting interferon.

In some embodiments, the "interferon-based therapeutic agent" includes multiple types of interferons or mutants or derivatives thereof.

The term "interferon-based therapeutic agent" covers various therapeutic agents comprising interferons or mutants or derivatives thereof that have been approved for marketing. In some embodiments, the "interferon-based therapeutic agent" is Pegberon (peginterferon α 2b, Amoytop). In some embodiments, the "interferon-based therapeutic agent" is Pegasys (peginterferon α 2a, Roche). In some embodiments, the "interferon-based therapeutic agent" is Pegintron (peginterferon α 2b injection, Schering-Plough). In some embodiments, the "interferon-based therapeutic agent" is Infergen (recombinant integrated interferon α, Amgen, USA). In some embodiments, the "interferon-based therapeutic agent" is Intron A (recombinant human interferon α 2b, Schering-Plough). In some embodiments, the "interferon-based therapeutic agent" is Roferon-A (Interferon α 2a, Roche). In some embodiments, the "interferon-based therapeutic agent" is Hapgen (recombinant human interferon α 1b, Beijing Sanyuan Jiyin Engineering Co., Ltd.) In some embodiments, the "interferon-based therapeutic agent" is PEGINFER (PEGlated-integrated interferon α-2 injection, Beijing Kawin Technology Co., Ltd.). In some embodiments, the "interferon-based therapeutic agent" is peginterferon λ (Nanogen Pharmaceutical biotechbology).

In some preferred embodiments, the interferon-based therapeutic agent includes interferon α 2b. In some preferred embodiments, the interferon-based therapeutic agent includes polyethylene glycol modified interferon α 2b. In some preferred embodiments, the interferon-based therapeutic agent is Pegberon.

In some embodiments, the "interferon-based therapeutic agent" includes an interferon or a mutant or derivative thereof, where the interferon or the mutant or derivative thereof includes an amino acid sequence of any one of SEQ ID NOs: 1-5, or the interferon or the mutant or derivative thereof includes an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to any one of SEQ ID NOs: 1-5.

In some embodiments, the "interferon-based therapeutic agent" also includes a nucleic acid molecule encoding an interferon or a mutant or derivative thereof, such as a recombinant nucleic acid expression vector. Suitable expression vectors, especially those suitable for therapeutic applications, may be easily determined by those skilled in the art.

The "interferon-based therapeutic agent" may also include a substance capable of promoting generation of endogenous interferons, such as agonists of the TLRs, RLRs, and STINGs signaling pathways. Examples of substances capable of promoting the generation of endogenous interferons include but are not limited to GS-9620, GS-9688, RO7020531, RO6864018, TQ-A3334, JNJ-4964, SB9200, MIW815, DMXAA, MK-1454, diABZI, an d the like^{[1]-[34]}.

As used herein, the term " consecutive treatment course" means that during the course of treatment, the administration of the therapeutic agent allows to continuously maintain the in vivo effective concentration of (exogenous or endogenous) interferon (for example, the effective blood concentration) within a subject, or to continuously maintain the in vivo concentration (for example, blood concentration) of neopterin (NPT), a main pharmacodynamic marker of interferons, within a subject to be higher than the concentration (initial concentration or baseline concentration) when the therapeutic agent is not administered. Since there is a good correlation between neopterin, a main pharmacodynamic marker of interferons, and the administration of interferons, it is particularly preferable to use the change in the in vivo concentration of neopterin (such as blood concentration) as the indicator of a "consecutive course of treatment". For example, a "consecutive course of treatment" may be defined as a course of treatment during which the administration of an interferon-based therapeutic agent for one or more times may keep the concentration of neopterin in a subject during substantially the entire course of treatment higher than the concentration of neopterin before the first administration (baseline concentration), for example approximately 110%, approximately 120%, approximately 130%, approximately 140%, approximately 150%, approximately 200%, approximately 250% or higher of the neopterin concentration before the first administration. The in vivo concentration of neopterin may be determined by methods known in the art.

In the consecutive treatment course, the administration scheme of the interferon-based therapeutic agent is generally determined by the characteristics of the selected therapeutic agent, such as its half-life in vivo. For example, in the consecutive treatment course, an long-acting interferon (with an in vivo half-life of generally 30-120 hours) may be administered about once a week, about once every two weeks, or may be administered once in a month or even a longer period in the case of increased dosage; and a short-acting interferon (with an in vivo half-life of generally 2-5 hours) may be administered once a day or once every two days or for three times a week, or may be administered once a week in the case of an increased dosage (for example, 9-36 MIU or higher), or may be administered for multiple times in a day, in the case of a reduced dosage. In the consecutive treatment course, the number of administrations of the interferon-based therapeutic agent is not particularly limited, as long as the above definition of the "consecutive treatment course" is met. Those skilled in the art may determine the consecutive treatment course based on the in vivo concentration (such as the blood concentration) of a pharmacodynamic marker such as neopterin of the interferon-based therapeutic agent.

In some embodiments, in the "consecutive treatment course", the "interferon-based therapeutic agent" may be administered in its conventional administration scheme. For example, Infergen (recombinant integrated interferon α), interferon α 2b (such as Intron A), interferon α 2a (such as Roferon-A), interferon α 1b (such as Hapgen) may be administered once a day or once every two days or for three times a week in the dosage range of 3-18 MIU. Peginterferon α 2a (e.g., Pegasys), peginterferon α 2b (e.g. Pegintron or Pegberon), PEGlated-integrated interferon α-2 (such as PEGINFER) or peginterferon λ may be administered once a week in the dosage range of 45-270 µg. P1101 may be administered in about 400 µg once every two weeks. The albuminated interferon α 2b may be administered at about 900 to about 1800 µg once every two weeks, or about 1200 µg once every 4 weeks.

The efficacy of interferon depends on the immune system. However, without being bound by any theory, it is believed that continuous administration of interferon for a long time will result in depletion of immune cells which will be difficult to recover, thereby reducing the efficacy. Therefore, the duration of the consecutive course should allow the therapeutic agent to achieve a certain effect but should avoid excessive depletion of immune cells. The depletion of immune cells in the treatment course is usually identified by changes in physiological/biochemical indicators.

In some embodiments, duration of each of the consecutive treatment courses is at least about 1 week.

In some embodiments, duration of each of the consecutive treatment courses is up to about 24 weeks.

In some embodiments, the duration of each of the consecutive treatment courses is about 1 week to about 24 weeks, for example, about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, or about 24 weeks.

In some preferred embodiments, the duration of each of the consecutive treatment courses is about 1 week to about 12 weeks. In some further preferred embodiments, the duration of each of the consecutive treatment courses is about 1 week to about 8 weeks. In some further preferred embodiments, the duration of each of the consecutive treatment courses is about 2 weeks to about 6 weeks. In some further preferred embodiments, the duration of each of the consecutive treatment courses is about 2 weeks.

In some embodiments, the end point of each consecutive treatment course (that is, the starting point of the interval between consecutive courses of treatment) may be the time of the last administration of the interferon-based therapeutic agent in the consecutive treatment course plus about 5 in vivo half-lives of the therapeutic agent. That is, the duration of the consecutive treatment course is the time period from the first administration to the last administration, plus about 5 in vivo half-lives of the therapeutic agent. It is believed that after 5 half-lives, the therapeutic agent will no longer generate a substantial recurrence preventing effect.

In the method of the present invention, the interval between the plurality of consecutive treatment courses may depend on the regeneration cycle of immune cells. The duration of the interval should allow the immune cells in the subject that have been reduced due to the administration of the agent to be restored to a level that can effectively implement the recurrence prevention. It is generally believed that it takes about 1-2 weeks for the immune cells to proliferate. Therefore, the shortest interval between the plurality of consecutive treatment courses can be about 1 week.

In some embodiments, the interval between the consecutive treatment courses is at least about 1 week.

In some embodiments, the interval between the consecutive treatment courses is up to about 24 weeks.

In some embodiments, the interval between the consecutive treatment courses is about 1 week to about 24 weeks, for example, about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, or about 24 weeks.

In some preferred embodiments, the interval between the consecutive treatment courses is about 1 week to about 12 weeks. In some more preferred embodiments, the interval between the consecutive treatment courses is are about 1 week to about 8 weeks. In some further preferred embodiments, the interval between the consecutive treatment courses is about 2 weeks to about 6 weeks.

In some embodiments, the duration of each of the consecutive treatment courses is about 1 week to about 24 weeks, and the interval between the treatment courses is about 1 week to about 24 weeks.

In some embodiments, the duration of each of the consecutive treatment courses is about 1 week to about 12 weeks, and the interval between the treatment courses is about 1 week to about 12 weeks.

In some embodiments, the duration of each of the consecutive treatment courses is about 1 week to about 8 weeks, and the interval between the treatment courses is about 1 week to about 8 weeks.

In some embodiments, the duration of each of the consecutive treatment courses is about 2 week to about 6 weeks, and the interval between the treatment courses is about 2 week to about 6 weeks.

In some embodiments, the duration of each of the consecutive treatment courses is about 2 weeks, and the interval between the treatment courses is about 2 weeks.

In some embodiments, the "interferon-based therapeutic agent" is administered for at least 2, at least 3, at least 4, at least 5, at least 6, at least 7, at least 8, at least 9, at least 10, at least 11, at least 12, at least 13, at least 14, at least 15, at least 20, at least 25 or more consecutive treatment courses.

In some embodiments, the durations of the plurality of consecutive treatment courses are substantially the same.

In some embodiments, the time intervals between the treatment courses are substantially the same.

In some specific embodiments, the interferon-based therapeutic agent is Pegberon, and the duration of each of the consecutive treatment courses is about 5 weeks to about 24 weeks, and the interval between the consecutive treatment courses is about 2 weeks to 8 weeks.

In some embodiments, the cancer includes, but is not limited to, leukemia (such as acute lymphocytic leukemia (ALL), acute myeloid leukemia (AML), chronic myeloid leukemia (CML), chronic lymphocytic leukemia, polycapillary leukemia), liver cancer, lung cancer, colorectal cancer, skin cancer, stomach cancer, breast cancer, prostate cancer, non-Hodgkin's lymphoma, melanoma, multiple myeloma, laryngeal papilloma, follicular lymphoma, AIDS-related Kaposi's sarcoma, renal cell carcinoma, and the like. In some embodiments, the disease is myeloproliferative tumor (MPN). In some preferred embodiments, the disease is liver cancer, lung cancer, breast cancer, colorectal cancer or melanoma.

In some embodiments, the method further comprises administering to the subject an additional anticancer agent. The additional anticancer agent is preferably used to prevent recurrence of cancer in the subject.

In some embodiments, the additional anticancer agent is a chemotherapeutic agent, including but not limited to, such as an alkylating agent: Nimustine, Carmustine, Lomustine, Cyclophosphamide, Ifosfamide, glyciphosphoramide, semustine; such as a chemotherapeutic antimetabolite: deoxyfluoguanosine, doxifluguanidine, 5-fluorouracil, mercaptopurine, thioguanine, cytarabine, fluguanosine, tegafur, Gemcitabine, carmofur, hydroxyurea, methotrexate, UFT, Ancitabine, capecitabine; such as a chemotherapeutic anti-tumor antibiotic: actinomycin D, doxorubicin, daunorubicin, Epirubicin, mitomycin, pelomycin, pingyangmycin, pirarubicin; such as a chemotherapeutic anti-tumor animal and plant ingredient: irinotecan, harringtonine, hydroxycamptothecin, Vinorelbine, paclitaxel, albumin paclitaxel, taxotere, topotecan, vincristine, vindesine, Vindesine, vinblastine, teniposide, etoposide, elemene; such as anti-tumor drug hormones: Atamestane, Anastrozole, Aminoglutethimide, Letrozole, Formestane, Metasterone, Tamoxifen; such as a chemotherapeutic miscellaneous agent: asparaginase, carboplatin, cisplatin, Dacarbazine, Oxaliplatin, Loxadine, Eloxatin, Mitoxantrone, or Procarbazine.

In some embodiments, the additional anticancer agent is a small molecule targeting drug, including but not limited to imatinib, gefitinib, bortezomib, erlotinib, sorafenib, lenalidomide, Sunitinib, dasatinib, nilotinib, lapatinib, pazopanib, everolimus, vandetanib, crizotinib, verofinib, ruxolitinib, axitinib, vismodegib, carfilzomib, regorafenib, bosutinib, tofacitinib, carbotinib, panatinib, pomalidomide, trametinib, dabrafenib, Afatinib, Icotinib, Ibrutinib, Ceritinib, Idelaris, Apatinib, Pabuccilib, Levatinib, Axitinib, Icotinib , Apatinib, sonidegib, cobimetinib, osimertinib, alectinib, ixazomib.

The additional anticancer agent may also be a tumor-associated antigen-specific antibody such as Rituxan, Herceptin and the like.

The additional anticancer agent may also be an immune checkpoint inhibitor, such as an inhibitor of PD1, PD-L1, CTLA4, etc., such as a specific antibody. Examples of an immune checkpoint inhibitor include, but are not limited to, Nivolumab, Pembrolizumab, Atezolizumab, Durvalumab, Avelumab and the like.

In some specific embodiments, the chemotherapeutic agent is oxaliplatin. In some specific embodiments, the chemotherapeutic agent is epirubicin. In some specific embodiments, the chemotherapeutic agent is paclitaxel.

In some preferred embodiments, the chemotherapeutic agent is an antimetabolitic chemotherapeutic agent, such as gemcitabine, capecitabine, and ancitabine. In some most preferred embodiments, the chemotherapeutic agent is gemcitabine.

In some embodiments, the administration of the "interferon-based therapeutic agent" does not overlap with the administration of the additional anticancer agent.

In some embodiments, the additional anticancer agent is administered between the plurality of consecutive treatment courses. For example, the additional anticancer agent may be administered for a period of time during the administration interval of the "interferon-based therapeutic agent", said period of time shorter than or equal to the interval.

In some embodiments, the administration of the "interferon-based therapeutic agent" overlaps with the administration of the additional anticancer agent.

In some embodiments, the additional anticancer agent is administered during and between the plurality of consecutive treatment courses, that is, the additional anticancer agent is administered during the entire preventive treatment period (completely overlapped).

In some embodiments, the additional anticancer agent is administered according to a conventional scheme, for example, its conventional administration scheme for the specific cancer being treated.

In one aspect, the present invention provides a pharmaceutical composition comprising an interferon-based therapeutic agent for use in preventing recurrence of cancer in a subject by a method of the present invention. In some embodiments, the pharmaceutical composition also comprises an additional anticancer agent. The interferon-based therapeutic agent and the anticancer agent are as defined above.

In one aspect, the present invention provides a pharmaceutical combination for use in preventing recurrence of cancer in a subject by the method of the invention, comprising an interferon-based therapeutic agent and an additional anticancer agent.

In one aspect, the present invention provides the use of an interferon-based therapeutic agent in preparation of a pharmaceutical composition for preventing recurrence of cancer in a subject by a method of the present invention. In some embodiments, the pharmaceutical composition also comprises an additional anticancer agent. The interferon-based therapeutic agent and the anticancer agent are as defined above.

In one aspect, the present invention provides the use of an interferon-based therapeutic agent in preparation of a pharmaceutical composition for preventing recurrence of cancer in a subject in combination with an additional anticancer agent. The interferon-based therapeutic agent and the anticancer agent are as defined above. The interferon-based therapeutic agent and/or the additional anticancer agent may be administered according to the method of the present invention.

In one aspect, the present invention provides a kit including an interferon-based therapeutic agent, and instructions for use, where the instructions for use provide a description that the interferon-based therapeutic agent may be used according to the method of the present invention or be administered according to the method of the present invention to prevent recurrence of cancer in a subject. In some embodiments, the kit further comprises an additional anticancer agent. The interferon-based therapeutic agent and the additional anticancer agent are as defined above.

### Examples

The following examples are only provided for better explaining the present invention and are not intended to limit the present invention in any way.

### Example 1. Preparation of recombinant mouse interferon α4 (mIFN-α4)

Interferons are also widely used in cancer treatment, but the effect needs to be improved. In order to study whether the continuous administration of interferon actually causes partial immune suppression and exhaustion of immune cells, which leads to poor subsequent treatment effects, the efficacy of interferon intermittent administration and continuous administration was compared in mice.

A large number of studies on anti-tumor effects were carried out in nude mice (lack of normal thymus) as the host. As the immune status of nude mice is weaker than that of normal mice, it is difficult to reflect the immune response under the normal state. In the anti-tumor model based on normal mice, the effect of interferon on the immune system may be partially realized, which reflects the effect of the anti-tumor therapy of administering the interferon. From the perspective of function realization, interferon should rely on the host's immune system to play its full anti-viral and anti-tumor effects. Because interferon has strong species specificity, when normal mice were used as the research subjects in the examples, the use of murine interferon or derivatives thereof may better reflect its physiological effects and implementation effects. In the research model using the mice of the present invention, PEGylated recombinant mouse interferon α4 (PEG-mIFNα4) was used as a representative of interferon therapeutic drugs and their derivatives.

According to the amino acid sequence of mIFN-α4 (GenBank NP_034634), the cDNA sequence of mIFN-α4 was optimized and designed according to the preferred codons of Pichia pastoris, and GenScript Biotechnology Co., Ltd. was entrusted to synthesize the cDNA. The cDNA encoding mIFN-α4 was recombined into pPIC9K plasmid, transformed into TOP10 competent cells, plated on LB solid medium, and cultured overnight at 37°C. A single clone was picked to inoculate the LB liquid medium, which was then cultured overnight at 37°C. The plasmid was extracted, and double digestion was performed with XhoI and NotI. Positive clones were identified by nucleic acid electrophoresis, and further confirmed by nucleic acid sequencing. The positive clone plasmid was digested and linearized with SalI, electrotransformed into Pichia pastoris GS115, plated on the RD plate, and cultured at 28-30°C for 3 days. The positive transformants were picked to inoculate YPD liquid medium, which was cultured overnight at 28-30°C, and transfered to BMMY medium at a final concentration of OD600nm of about 1, and incubated at 28-30°C for about 24 hours. Methanol was added until the final concentration of methanol of about 1 %, and then further cultured at 28-30 °C for about 24 hours. The culture medium was centrifuged to collect the supernatant, and the expression of mIFN-α4 was detected by SDS-PAGE electrophoresis. According to the results of SDS-PAGE electrophoresis, the engineered strains with higher expression and stable expression were selected for subsequent fermentation in a fermenter.

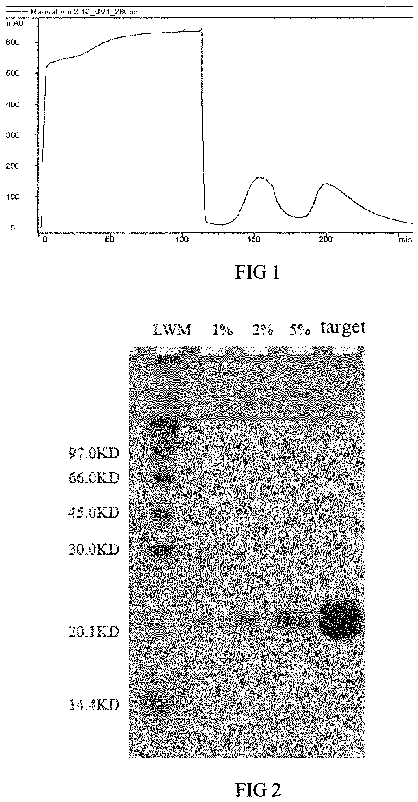

The fermenter was 30L. Refer to the "Pichia Fermentation Process Guidelines" for fermentation culture and methanol induction, the induction time was about 30h. The fermentation supernatant was collected by centrifugation, and concentrated by ultrafiltration for 3~5 times in 5K hollow fiber membrane tube, and the buffer system was replaced with 20mM phosphate buffer-20mM arginine hydrochloride-50mM sodium chloride buffer solution (pH6.5). SP Sepharose Fast Flow chromatography column (GE Healthcare, column bed Φ38mm×160mm) was then loaded, 20mM phosphate buffer -20mM arginine hydrochloride (pH6.5) (solution A) was then used to wash about 3 column volumes. The solution A and 20mM phosphate buffer -20mM arginine hydrochloride - 1M sodium chloride solution (pH6.5) (solution B) were used to perform gradient elution. mIFN-α4 target samples were collected, and sample for non-reducing SDS-PAGE (with the separation gel concentration of 14%, silver staining) were taken. The elution profile is shown in FIG. 1, and the electrophoresis result is shown in FIG. 2.

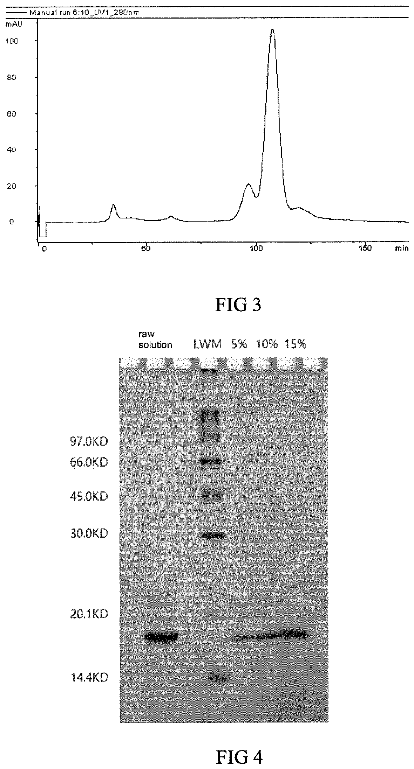

mIFN-α4 SP Sepharose Fast Flow purification sample was concentrated by ultrafiltration with 5K ultrafiltration membrane package and replaced with 20mM phosphate buffer-50mM arginine hydrochloride-10mM methionine-20mM sodium chloride (pH 7.0), and then its concentration was adjusted to about 1.0mg/ml. Glycosidase was added at a mass ratio of mIFN-α4 protein to enzyme of about 20:1, and digestion was performed at 25°C for about 20 hours to remove glycosyl groups. The digested sample was diluted by about 6 times with SmM boric acid buffer-10mM arginine hydrochloride (pH9.0), and the sample was loaded on Q Sepharose Fast Flow chromatography column (GE Healthcare, column bed Φ50mm×154mm). 20mM boric acid buffer -20mM arginine hydrochloride-lOmM methionine (pH 9.0) (solution C) was used to wash about 3 column volumes. The solution C and 20mM boric acid buffer -20mM arginine hydrochloride-lOmM formazan thionine-0.3M sodium chloride (pH 9.0) (solution D) were used for gradient elution. The target deglycosylated mIFN-α4 sample was collected, and the pH was adjusted to about pH 5.0 with 10% acetic acid. 5K ultrafiltration membrane was used to concentrate by ultrafiltration and the buffer was replaced with 5mM acetic acid/sodium acetate buffer-50mM arginine hydrochloride-100mM sodium chloride (pH 5.0). The resulting sample was the stock solution of deglycosylated mIFN-α4. Samples were taken to be sent for inspection, and the remaining samples were frozen at -70°C for later use. The elution profile is shown in FIG. 3, and the SDS-PAGE electrophoresis result is shown in FIG. 4.

The bacterial endotoxin content of the deglycosylated mIFN-α4 stock solution was determined by the Limulus reagent method as lower than 60 EU/mg. specific activity was determined as 5.4×10⁸U/mg by using the commercial mIFN-α4 (R&D, catalog number 12115-1) as the standard, and by using the mouse fibroblast/encephalomyocarditis virus (L929/EMCV) cytopathic inhibition method.

### Example 2. Preparation of PEGed recombinant mouse interferon (PEG-mIFN-α4)

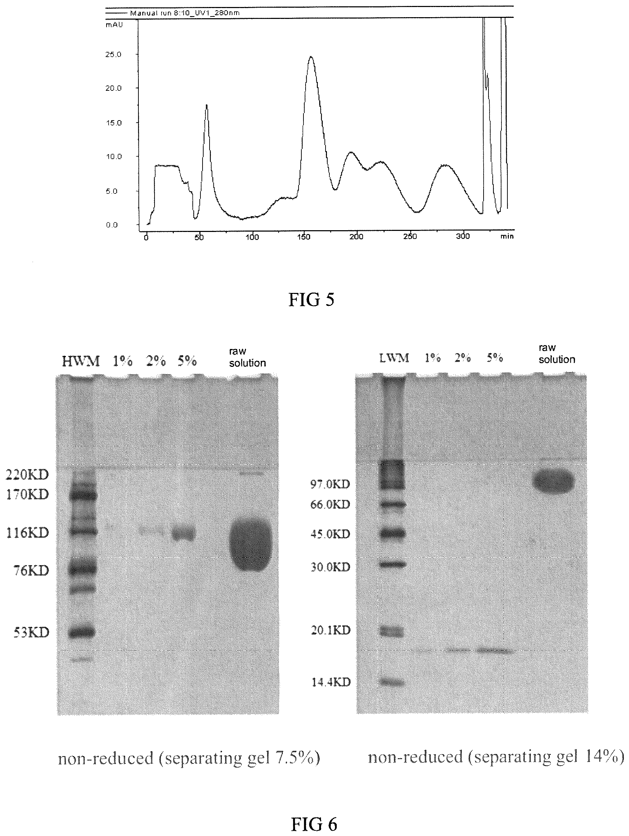

The deglycosylated mIFN-α4 stock solution was replaced through ultrafiltration with buffer of 5 mM acetic acid/sodium acetate buffer-50mM sodium chloride (pH5.0) with 5K ultrafiltration membrane package. About 333m1 sample (with deglycosylated mIFNα4 content of about 500mg) was taken, added with about 22ml of 0.8M boric acid/sodium hydroxide buffer (pH 9.4), and stirred well. YPEG-NHS was added based on the mass ratio of protein to 40kD Y-type polyethylene glycol succinimide ester (YPEG-NHS) of about 1:8, which was then stirred quickly, and reacted at room temperature for 10 minutes. Then about 20ml of 0.2M methionine was added to stop the reaction, and the pH was adjusted to 5.0 with 10% acetic acid. 550ml pure water was then added, and then 600ml 20mM acetic acid/sodium acetate buffer-20mM arginine hydrochloride-10mM methionine (pH5.1) (solution E), mixed well. After that, it was loaded on the SP Sepharose Fast Flow chromatography column (GE Healthcare, column bed Φ50mm×194mm), and the solution E was used to wash about 5 column volumes. After that, the solution E and 20mM acetic acid/sodium acetate buffer-20mM arginine hydrochloride-10mM methionine-600mM Sodium chloride (pH5.1) (solution F) were used for gradient elution, and PEG-mIFN-α4 target samples were collected. The buffer was replaced through ultrafiltration by using the 5K ultrafiltration membrane package to 20mM phosphate buffer-123mM sodium chloride (pH 6.5). and then concentrated appropriately, added with 0.5% Tween80 to the final concentration of Tween80 being about 0.005%. The resulting sample was the PEG-mIFN-α4 stock solution (PEG-mIFN-α4). Samples were taken and then sent for inspection, the remaining samples were frozen and stored at -70°C for later use. The elution profile is shown in FIG. 5, and the SDS-PAGE electrophoresis result is shown in FIG. 6.

The bacterial endotoxin content of the PEG-mIFN-α4 stock solution was determined by the Limulus reagent method as lower than 15 EU/mg. Specific activity was determined as 6.1×10⁶U/mg by using the commercial mIFN-α4 (R&D, catalog number 12115-1) as the standard, and by using the mouse fibroblast/encephalomyocarditis virus (L929/EMCV) cytopathic inhibition method.

### Example 3. Pharmacokinetic study of subcutaneous injection of PEG-mIFN-α4 in healthy BALB/c mice

In this example, BALB/c mice were purchased from Beijing Vital River Laboratory Animal Technology Co., Ltd. The blood concentration of PEG-mIFN-α4 was detected by double-antibody sandwich ELISA. The test kit of the Mouse IFN alpha Platinum ELISA kit (Cat. No. BMS6027/ BMS6027TEN, Thermo) was used, but the reporter antibody was replaced with the anti-PEG antibody 3.3 biotin (Institute of Biomedical Sciences, Academia Sinica (IBMS), Taiwan, China) to avoid interference of endogenous mIFN-α on the measurement result.

6-8 weeks old, SPF grade BALB/c mice (N=65) were injected with a single injection of 1 µg/mouse PEG-mIFN-α4 subcutaneously on the back of the neck. Plasma samples were collected before the injection (0h) and after the injection at 15h, 24h, 36h, 48h, 72h, 96h, 120h, 168h, 216h, 264h, 312h, to determine the blood concentration. Phoenix WinNonlin 6.4 software was used to calculate the pharmacokinetic parameters.

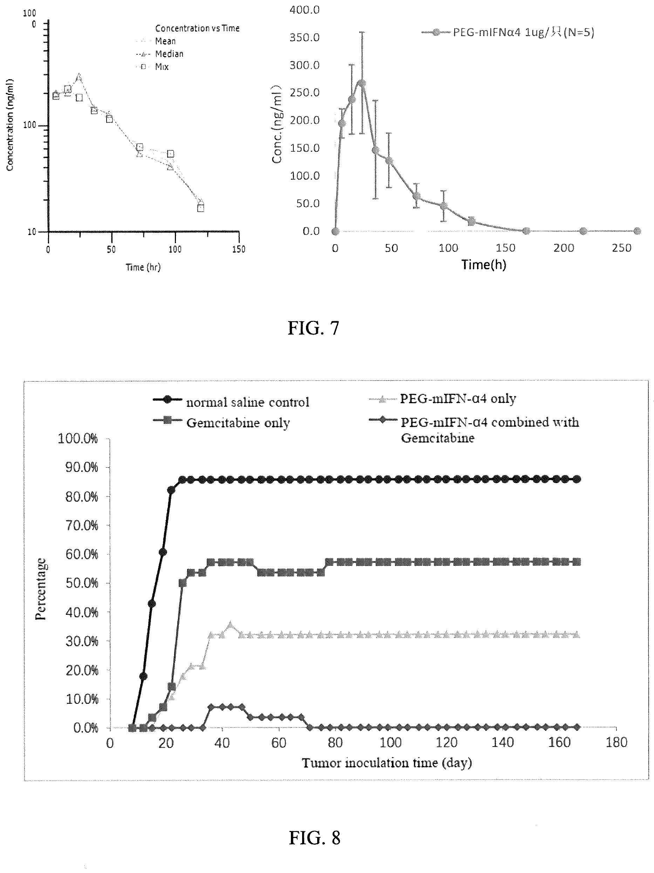

The results of blood concentration determination are shown in Table 1, the blood concentration-administration time curve is shown in FIG. 7, and the results of pharmacokinetic parameters are shown in Table 2. With a single injection of 1µg/mouse of PEG-mIFN-α4 subcutaneously on the back of the neck of BALB/c mice, plasma concentration peak time (Tₘₐₓ) was 24h, peak concentration time (Tₘₐₓ) was 268ng/ml, and elimination half-life (T_{1/2}) was 28.3h.

**Table 1. Results of blood concentration determination of BALB/c mice with a single subcutaneous injection of 1µg/mouse of PEG-mIFN-α4 (N=5, ng/ml)**

| **Collected at** | **blood concentration (ng/ml)** | | | | | **Mean (ng/ml)** | **SD** |
|---|---|---|---|---|---|---|---|
| | **1** | **2** | **3** | **4** | **5** | | |
| 0h | 0 | 0 | 0 | 0 | 0 | 0.0 | 0.0 |
| 6h | 218.1 | 165.9 | 167.8 | 220.8 | 200.6 | 194.6 | 26.5 |
| 15h | 282.7 | 326.1 | 194.3 | 203.8 | 183.2 | 238.0 | 62.9 |
| 24h | 341.5 | 290.8 | 362.8 | 185.3 | 159.7 | 268.0 | 91.5 |
| 36h | 204.8 | 263.1 | 145.4 | 60.6 | 61.4 | 147.1 | 88.9 |
| 48h | 205.8 | 128.1 | 133.3 | 79 | 91.4 | 127.5 | 49.5 |
| 72h | 95.5 | 73.6 | 40.4 | 52.9 | 54.6 | 63.4 | 21.5 |
| 96h | 41.2 | 52.6 | 87.5 | 20.6 | 22.2 | 44.8 | 27.4 |
| 120h | 22.6 | 26.1 | 19.1 | 11.5 | 10.6 | 18.0 | 6.8 |
| 168h | BLQ | BLQ | BLQ | BLQ | BLQ | BLQ | / |
| 216h | BLQ | BLQ | BLQ | BLQ | BLQ | BLQ | / |
| 264h | BLQ | BLQ | BLQ | BLQ | BLQ | BLQ | / |
| 312h | BLQ | BLQ | BLQ | BLQ | BLQ | BLQ | / |

| | | | | | | | |
|---|---|---|---|---|---|---|---|
| Note: In the pharmacokinetic analysis, for samples with blood concentration lower than the quantification lower limit, the sample before the peak blood concentration was counted as 0, and the sample after the peak was expressed as BLQ. | | | | | | | |

**Table 2. Summary of pharmacokinetic parameters of a single subcutaneous injection of 1 µg/mouse of PEG-mIFN-α4 in BALB/c mice**

| **Parameter** | **Unit** | PEG-mIFNα4 |
|---|---|---|
| T_{1/2} | hr | **28.3** |
| Tₘₐₓ | hr | 24.0 |
| Cₘₐₓ | ng/ml | 268.0 |
| Tₗₐₛₜ | hr | 120.0 |
| AUCₗₐₛₜ | hr*ng/ml | 13289.3 |
| AUCINF_{_obs} | hr*ng/ml | 14023.2 |
| Vz_F_{_obs} | ml | 2.91 |
| C1_F_{_obs} | ml/hr | 0.07 |

### Example 4. Comparative study on the prevention of recurrence of transplanted liver cancer H22 in mice by intermittent administration of PEG-mIFN-α4, or intermittent administration of PEG-mIFN-α4 combined with gemcitabine administration

In this example, BALB/c mice were purchased from Beijing Vital River Laboratory Animal Technology Co., Ltd., and the mouse liver cancer H₂₂ cell line was purchased from the China Center for Type Culture Collection (CCTCC).

After tumor treatment, the tumor load in patient's body is relatively low, so a low tumor grafting volume is used to simulate the state after tumor treatment. 6-8 weeks old, 18-22g SPF grade BALB/c mice were subcutaneously inoculated with H22 cells (1×10⁴/mL, 0.2ml/mouse) in the armpit of the right forelimb. Three days after tumor inoculation, they were randomly divided into the following experiment groups: PEG-mIFN-α4 group (N=28, 14 females, 14 males), PEG-mIFN-α4 combined with gemcitabine group (N=28, 14 females, 14 males), normal saline control group (N=28, 14 females, 14 males), and gemcitabine only group (N=28, 14 females, 14 males).

PEG-mIFN-α4 was administered by subcutaneous injection on the back of the neck, with a dose of 1 microgram per mouse. Administration began on the day after grouping. In the PEG-mIFN-α4 group, PEG-mIFN-α4 was administered once a week for 9 weeks, and then stopped for 103 days. In the PEG-mIFN-α4 combined with gemcitabine group, PEG-mIFN-α4 and gemcitabine were administered once a week for 9 weeks, and then stopped for 103 days. In the gemcitabine alone group, gemcitabine was administered once a week for 9 weeks, and then stopped for 103 days. The normal saline control group was given an equal volume of normal saline. Gemcitabine was injected intraperitoneally at a dose of 60 mg/kg.

The tumor incidence of each group was calculated when the tumor volume was greater than or equal to 62.5mm³, which was determined as positive for tumor occurrence. The changes in tumor incidence are shown in Figure 8. By day 166 after tumor inoculation, the tumor incidence in the normal saline control group was 85.7%, that in the PEG-mIFN-α4 only group was 32.1%, that in the gemcitabine only group was 57.1%, and that in the PEG-mIFN-α4 combined with gemcitabine group was 0.0%. Mice that were determined to be negative for tumor occurrence were dissected and confirmed, and no solid tumors were found at the tumor site and its vicinity. The comparison results of tumor incidence in each group are shown in Table 3. The tumor incidence of the PEG-mIFN-α4 only group and the PEG-mIFN-α4 combined with gemcitabine group were lower than that of normal saline control group; the tumor incidence of the PEG-mIFN-α4 combined with gemcitabine group was lower than that of PEG-mIFN-α4 only group and gemcitabine only group. PEG-mIFN-α4 combined with gemcitabine group was superior to PEG-mIFN-α4 only group and gemcitabine only group.

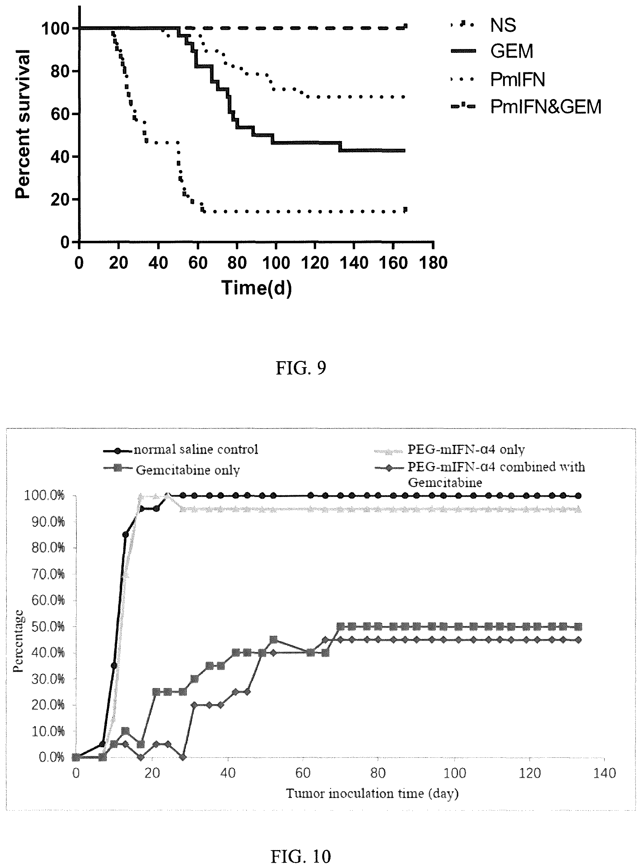

The survival curves is shown in Figure 9, and the results of the median survival period of each group are shown in Table 4. By day 166 after tumor inoculation, the median survival time of the normal saline control group was 33.5 days, and that of the gemcitabine alone group was 93.0 days. The survival rate of the PEG-mIFN-α4 alone group was 67.9%, and that of the PEG-mIFN-α4 intermittent administration combined with gemcitabine group was 100.0%. Compared with the gemcitabine only group and the PEG-mIFN-α4 only group, the survival time of the PEG-mIFN-α4 combined with gemcitabine group was prolonged. PEG-mIFN-α4 combined with gemcitabine group was superior to PEG-mIFN-α4 only group and gemcitabine only group.

The results showed that for recurrence of H22 transplanted liver cancer in mice simulated with low-density tumor inoculation, intermittent administration of pegylated murine interferon α combined with gemcitabine, administration of gemcitabine only, and administration of pegylated murine interferon α only all had anticancer activity and could effectively prevent tumor recurrence, and pegylated murine interferon α combined with gemcitabine group was superior to administration of gemcitabine only or pegylated murine interferon α only.

**Table 3. The tumor incidence results of the comparative study on the prevention of recurrence of transplanted liver cancer H22 in mice by administration of PEG-mIFN-α4 only or administration of PEG-mIFN-α4 combined with gemcitabine**

| **Group** | **No. of Animals (female+male)** | **Tumor incidence** |
|---|---|---|
| normal saline control | 14+14 | 85.7% |
| PEG-mIFN-α4 only | 14+14 | 32.1% |
| Gemcitabine only | 14+14 | 57.1% |
| PEG-mIFN-α4 combined with gemcitabine | 14+14 | 0.0% |

**Table 4. The median survival time results of the comparative study on the prevention of recurrence of transplanted liver cancer H22 in mice by administration of PEG-mIFN-α4 alone or administration of PEG-mIFN-α4 combined with gemcitabine**

| **Group** | **No. of Animals (female+male)** | **Median survival time (time after tumor inoculation, days)** |
|---|---|---|
| normal saline control | 14+14 | 33.5 |
| PEG-mIFN-α4 only | 14+14 | survival rate during the observation period: 67.9% |
| Gemcitabine only | 14+14 | 93.0 |
| PEG-mIFN-α4 combined with gemcitabine | 14+14 | survival rate during the observation period: 100.0% |

### Example 5. Comparative study on the prevention of recurrence of transplanted colorectal cancer CT26 in mice by intermittent administration of PEG-mIFN-α4, or intermittent administration of PEG-mIFN-α4 combined with gemcitabine administration

In this example, BALB/c mice were purchased from Beijing Vital River Laboratory Animal Technology Co., Ltd., and the mouse colorectal cancer CT26 cell line was purchased from the Cell Resource Center of Shanghai Academy of Biological Sciences, Chinese Academy of Sciences.

6-8 weeks old, 18-22g SPF grade BALB/c mice were subcutaneously inoculated with CT26 cells (1×10⁵ cells/mL, 0.2ml/mouse) in the armpit of the right forelimb. On the day after tumor inoculation, they were randomly divided into the following experiment groups: PEG-mIFN-α4 only group (N=20, 10 females and 10 males), normal saline control group (N=20, 10 females and 10 males), gemcitabine only group (N=20, 10 females and 10 males), and PEG-mIFN-α4 combined with gemcitabine group (N=20, 10 females and 10 males).

PEG-mIFN-α4 was administered by subcutaneous injection on the back of the neck, with a dose of 1 microgram per mouse. Administration began on the day after grouping. In the PEG-mIFN-α4 only group, PEG-mIFN-α4 was administered once a week for 12 times, and then stopped for 56 days. In the PEG-mIFN-α4 combined with gemcitabine group, both PEG-mIFN-α4 and gemcitabine were administered once a week for 12 consecutive times, and then stopped for 56 days. In the gemcitabine only group, gemcitabine was administered once a week for 12 times, and then stopped for 56 days. The normal saline control group was given an equal volume of normal saline. Gemcitabine was administered by intraperitoneal injection at a dose of 60 mg/kg.

The tumor incidence of each group was calculated when the tumor volume was greater than or equal to 62.5mm³, which was determined as positive for tumor occurrence. The changes in tumor incidence are shown in Figure 10. By day 134 after tumor inoculation, the tumor incidence in the normal saline control group was 100%, that in the PEG-mIFN-α4 only group was 95%, that in the gemcitabine only group was 50%, and that in the PEG-mIFN-α4 combined with gemcitabine group was 45%. The comparison results of tumor incidence in each group are shown in Table 5. The tumor incidence of the PEG-mIFN-α4 only group and the PEG-mIFN-α4 combined with gemcitabine group were lower than that of normal saline control group; the tumor incidence of the PEG-mIFN-α4 combined with gemcitabine group was lower than that of PEG-mIFN-α4 only group and gemcitabine only group. PEG-mIFN-α4 combined with gemcitabine group was superior to PEG-mIFN-α4 only group and gemcitabine only group.

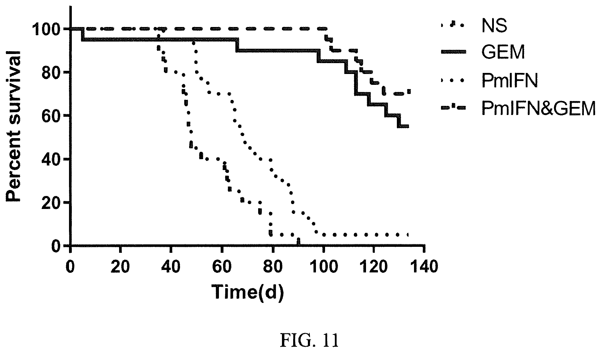

The survival curves are shown in Figure 11, and the results of the median survival period of each group are shown in Table 6. By day 134 after tumor inoculation, the median survival time of the normal saline control group was 48.0 days, and the survival rate of the gemcitabine alone group was 55.0%. The median survival time of the PEG-mIFN-α4 alone group was 68.5 days, and the survival rate of the PEG-mIFN-α4 intermittent administration combined with gemcitabine group was 70.0%. Compared with the gemcitabine only group and the PEG-mIFN-α4 only group, the survival time of the PEG-mIFN-α4 combined with gemcitabine group was prolonged. PEG-mIFN-α4 combined with gemcitabine group was superior to PEG-mIFN-α4 only group and gemcitabine only group.

The results showed that for recurrence of CT26 transplanted colorectal cancer in mice simulated with low-density tumor inoculation, intermittent administration of pegylated murine interferon α combined with gemcitabine, administration of gemcitabine only, and administration of pegylated murine interferon α only all had anticancer activity and could effectively prevent tumor recurrence, and pegylated murine interferon α combined with gemcitabine group was superior to administration of gemcitabine only or pegylated murine interferon α only.

**Table 5. The tumor incidence results of the comparative study on the prevention of recurrence of CT26 transplanted colorectal cancer in mice by administration of administration of PEG-mIFN-α4 combined with gemcitabine**

| **Group** | **No. of Animals (female+male)** | Tumor incidence |
|---|---|---|
| normal saline control | 10+10 | 100.0% |
| PEG-mIFN-α4 only | 10+10 | 95.0% |
| Gemcitabine only | 10+10 | 50.0% |
| PEG-mIFN-α4 combined with gemcitabine | 10+10 | 45.0% |

**Table 6. The median survival time results of the comparative study on the prevention of recurrence of CT26 transplanted colorectal cancer in mice by administration of administration of PEG-mIFN-α4 combined with gemcitabine**

| **Group** | **No. of Animals (female+male)** | **Median survival time (time after tumor inoculation, days)** |
|---|---|---|
| normal saline control | 10+10 | 48.0 |
| PEG-mIFN-α4 only | 10+10 | 68.5 |
| Gemcitabine only | 10+10 | survival rate during the observation period: 55.0% |
| PEG-mIFN-α4 combined with gemcitabine | 10+10 | survival rate during the observation period: 70.0% |

### Sequences and description thereof

>SEQ ID NO: 1 is the amino acid sequence of interferon α 2a (e.g., Roferon-A or Pegasys) 
CDLPQTHSLGSRRTLMLLAQMRKISLFSCLKDRHDFGFPQEEFGNQFQKAETIPVLHE
MIQQIFNLFSTKDSSAAWDETLLDKFYTELYQQLNDLEACVIQGVGVTEPLMKEDSILAVR
KYFQRITLYLKEKKYSPCAWEVVRAEIMRSFSLSTNLQESLRSKE
>SEQ ID NO: 2 is the amino acid sequence of interferon α 2b (e.g., INTRONA or PEG-INTRON or Pegberon)
>SEQ ID NO: 3 is the amino acid sequence of a recombinant integrated interferon (e.g., Infergen) 
MCDLPQTHSLGNRRALILLAQMRRISPFSCLKDRHDFGFPQEEFDGNQFQKAQAISV
LHEMIQQTFNLFSTKDSSAAWDESLLEKFYTELYQQLNDLEACVIQEVGVEETPLMNVDSI
LAVKKYFQRITLYLTEKKYSPCAWEVVRAEIMRSFSLSTNLQERLRRKE
>SEQ ID NO: 4 is the amino acid sequence of integrated interferon α-2 (e.g., PEGINFER) 
GSGGGCDLPQTHSLGNRRALILLAQMRRISPFSCLKDRHDFGFPQEEFDGNQFQKAQ
AISVLHEMIQQTFNLFSTKDSSAAWDESLLEKFYTELYQQLNDLEACVIQEVGVEETPLMN
VDSILAVRKYFQRITLYLTEKKYSPCAWEVVRAEIMR SFSLSTNLQERLRRKD
>SEQ ID NO: 5 is the amino acid sequence of interferon λ 
MGPVPTSKPTTTGKGCHIGRFKSLSPQELASFKKARDALEESLKLKNWSCSSPVFPGN
WDLRLLQVRERPVALEAELALTLKVLEAAAGPALEDVLDQPLHTLHHILSQLQACIQPQPT
AGPRPRGRLHHWLHRLQEAPKKESAGCLEASVTFNLFRLLTRDLKYVADGNLCLRTSTHP
EST
>SEQ ID NO: 6 is the amino acid sequence of peginterferon murine α 4

CDLPHTYNLGNKRALTVLEEMRRLPPLSCLKDRKDFGFPLEKVDNQQIQKAQAILVL
RDLTQQILNLFTSKDLSATWNATLLDSFCNDLHQQLNDLKACVMQEPPLTQEDSLLAVRT
YFHRITVYLRKKKHSLCAWEVIRAEVWRALSSSTNLLARLSEEKE

### References:

[1] Gisslinger H, et. al. Ropeginterferon alfa-2b, a novel IFNα-2b, induces high response rates with low toxicity in patients with polycythemia vera. Blood. 2015 Oct 8;126(15):1762-9. doi: 10.1182/blood-2015-04-637280. Epub 2015 Aug 10.
[2] Gane EJ, et. al. The oral toll-like receptor-7 agonist GS-9620 in patients with chronic hepatitis B virus infection. J Hepatol. 2015 Aug;63(2):320-8. doi: 10.1016/j.jhep.2015.02.037. Epub 2015 Feb 27.
[3] Janssen HLA, et. al. Safety, efficacy and pharmacodynamics of vesatolimod (GS-9620) in virally suppressed patients with chronic hepatitis B. J Hepatol. 2018 Mar;68(3):431-440. doi: 10.1016/j.jhep.2017.10.027. Epub 2017 Dec 11.
[4] Agarwal K, et. al. Safety and efficacy of vesatolimod (GS-9620) in patients with chronic hepatitis B who are not currently on antiviral treatment. Journal of Viral Hepatitis [2018 Aug, 25(11):1331-1340.
[5] Lopatin U, et. al. Safety, pharmacokinetics and pharmacodynamics of GS-9620, an oral Toll-like receptor 7 agonist. Antivir Ther. 2013;18(3):409-18. doi: 10.3851/IMP2548. Epub 2013 Feb 15.
[6] Edward J. Gane, et. al. TLR7 agonist RO7020531 triggers immune activation after multiple doses in chronic hepatitis B patients Reported by Jules Levin. AASLD 2018 Nov 9-13 SF
[7] Ebrahim M, et. al. Are RIG-1 and MDA5 Expressions Associated with Chronic HBV Infection? Viral Immunol. 2015 Nov;28(9):504-8. doi: 10.1089/vim.2015.0056. Epub 2015 Oct 20.
[8] Ma Z, et. al. Contribution of Toll-like receptors to the control of hepatitis B virus infection by initiating antiviral innate responses and promoting specific adaptive immune responses. Cell Mol Immunol. 2015 May;12(3):273-82. doi: 10.1038/cmi.2014.112. Epub 2014 Nov 24.
[9] Jones M, et. al. SB 9200, a novel agonist of innate immunity, shows potent antiviral activity against resistant HCV variants. J Med Virol. 2017 Sep;89(9):1620-1628. doi: 10.1002/jmv.24809. Epub 2017 May 23.
[10] Xu CL, et. al. Upregulation of toll-like receptor 4 on T cells in PBMCs is associated with disease aggravation of HBV-related acute-on-chronic liver failure. J Huazhong Univ Sci Technolog Med Sci. 2015 Dec;35(6):910-915. doi: 10.1007/sll596-015-1527-x. Epub 2015 Dec 16.
[11] Kato H, et. al. RIG-I-Like Receptors and Type I Interferonopathies. J Interferon Cytokine Res. 2017 May;37(5):207-213. doi: 10.1089/jir.2016.0095.
[12] Li L, et. al. Anti-HBV response to toll-like receptor 7 agonist GS-9620 is associated with intrahepatic aggregates of T cells and B cells. J Hepatol. 2018 May;68(5):912-921. doi: 10.1016/j.jhep.2017.12.008. Epub 2017 Dec 14.
[13] Harrington, et. al. LBA15Preliminary results of the first-in-human (FIH) study of MK-1454, an agonist of stimulator of interferon genes (STING), as monotherapy or in combination with pembrolizumab (pembro) in patients with advanced solid tumors or lymphomas. Annals of Oncology.2018 OCT.29(8):712-712.Meeting Abstract.
[14] Cemerski, Saso, et. al. Preclinical characterization of a novel STING agonist, MK-1454. JOURNAL FOR IMMUNOTHERAPY OF CANCER. 2017 NOV.5(2):16.Meeting Abstract.
[15] Ramanjulu JM, et. al. Design of amidobenzimidazole STING receptor agonists with systemic activity. Nature. 2018 Dec;564(7736):439-443. doi: 10.1038/s41586-018-0705-y. Epub 2018 Nov 7.
[16] Safety and Efficacy of MIW815 (ADU-S100) +/- Ipilimumab in Patients With Advanced/Metastatic Solid Tumors or Lymphomas. https://www.aduro.com/file.cfm/13/docs/SITC18_MIW815X21011_SITC%20Poster(e_versio n)_08Nov2018.pdf
[17]Lara PN Jr, et. al. Randomized phase III placebo-controlled trial of carboplatin and paclitaxel with or without the vascular disrupting agent vadimezan (ASA404) in advanced non-small-cell lung cancer. J Clin Oncol. 2011 Aug 1;29(22):2965-71. doi: 10.1200/JCO.2011.35.0660. Epub 2011 Jun 27.
[18] Yuen, M. F, et. al. Dose response and safety of the daily, oral RIG-I agonist Inarigivir (SB 9200) in treatment naive patients with chronic hepatitis B: results from the 25mg and 50mg cohorts in the ACHIEVE trial. JOURNAL OF HEPATOLOGY. 2018 APR.68(1): S509-S510.DOI: 10.1016/S0168-8278(18)31267-4
[19] Ma Z, et. al. Interaction between Hepatitis B Virus and Toll-Like Receptors: Current Status and Potential Therapeutic Use for Chronic Hepatitis B. Vaccines (Basel). 2018 Jan 16;6(1). pii: E6. doi: 10.3390/vaccines6010006.
[20] Walsh, R., et. al. Effects of SB9200 (Inarigivir) therapy on immune responses in patients with chronic hepatitis B.JOURNAL OF HEPATOLOGY. 2018 APR.68(1): S89-S89. DOI: 10.1016/S0168-8278(18)30396-9
[21] Niu C, et. al. Toll-like receptor 7 agonist GS-9620 induces prolonged inhibition of HBV via a type I interferon-dependent mechanism. J Hepatol. 2018 May;68(5):922-931. doi: 10.1016/j.jhep.2017.12.007. Epub 2017 Dec 13.
[22] Yuen, MF, et. al. Ascending dose cohort study of inarigivir - A novel RIG I agonist in chronic HBV patients: Final results of the ACHIEVE trial. JOURNAL OF HEPATOLOGY. 2019 APR.70(1): E47-E48.DOI: 10.1016/S0618-8278(19)30084-2
[23] Bourquin C, et. al. Harnessing the immune system to fight cancer with Toll-like receptor and RIG-I-like receptor agonists. Pharmacol Res. 2019 Mar 2. pii: S1043-6618(18)32066-8. doi: 10.1016/j.phrs.2019.03.001.
[24] Zeng Y, et. al. Toll-like receptors, long non-coding RNA NEAT1, and RIG-I expression are associated with HBeAg-positive chronic hepatitis B patients in the active phase. J Clin Lab Anal. 2019 Jun;33(5):e22886. doi: 10.1002/jcla.22886. Epub 2019 Mar 29.
[25] Wang Jiawen, et al. Research progress of Toll-like receptors and their agonists. Advances in Physiological Science, 2018, Vol. 49, No. 4, 289-292.
[26] Tao Zeyu, et al. Research progress of drugs for the treatment of chronic hepatitis B virus infection. Foreign Medicine and Antibiotics Volume, July 2018, Vol. 39, No. 4: 308-319.
[27] Xu Qun, et al. Research progress of immunomodulators targeting Toll-like receptors. Advances in Pharmacy, 2016, 40(1): 42-55.
[28] Liu Qiuming, et al. New Advances in Anti-HBV Drug Research, Journal of Virology. September 2016, Vol. 32, Issue 5: 650-658.
[29] Mu Na, et al. Expression levels of pattern recognition receptors TLR3, RIG-I and MDA5 in peripheral blood of patients with chronic hepatitis B. Chinese Journal of Immunology. 2016-05:715-719.
[30] Heinz Gisslinger, et. al. Ropeginterferon alfa-2b, a novel IFNα-2b, induces high response rates with low toxicity in patients with polycythemia vera.Blood. 2015 Oct 8; 126(15): 1762-1769.Prepublished online 2015 Aug 10.
[31] Monkarsh SP, et. al. Positional isomers of monopegylated interferon alpha-2a: isolation, characterization, and biological activity. Anal Biochem. 1997 May 1;247(2):434-40.
[32] Grace M, et. al. Structural and biologic characterization of pegylated recombinant IFN-alpha2b. J Interferon Cytokine Res. 2001 Dec;21(12):1103-15.
[33] Foser S, et. al. Isolation, structural characterization, and antiviral activity of positional isomers of monopegylated interferon alpha-2a (PEGASYS). Protein Expr Purif. 2003 Jul;30(1):78-87.
[34] Nanogen Pharmaceutical biotechbology.PEG-INTERFERON LAMBDA 1 CONJUGATES.WO2013/028233 A1. 28.02.2013.
[35] Nanogen Pharmaceutical biotechbology.PEG-INTERFERON LAMBDA 1 CONJUGATES.US 8454947 B1.Jun.4.2013.
   https://worldwide.espacenet.com/searchResults?submitted=true&locale=en_EP&DB=EPOD OC&ST=advanced&TI=&AB=peginterferon+lambda&PN=&AP=&PR=&PD=&PA=&IN=&CPC=&IC=
[36] Wang Junzhi. Biotechnology drug research and development and quality control. 2nd edition. Science Press, 2007: 540.
[37] Interferon alpha-4 precursor [Mus musculus].https://www.ncbi.nlm.nih.gov/protein/NP_034634.
[38] Pichia Fermentation Process Guidelines, Thermo Fisher Scientific Inc.https://www.thermofisher.com/document-connect/document-connect.html?url=https% 3A%2F%2Fassets.thermofisher.com%2FTFS-Assets%2FLSG%2Fmanuals%2 Fpichiaferm_prot.pdf&title=UGljaGlhIEZlcm1lbnRhdGlvbiBHdWlkZWxpbmVz.

## Claims

1. A method for preventing cancer recurrence in a subject, comprising
i) administering an interferon-based therapeutic agent; and
ii) optionally, administering an additional anticancer agent,
to the subject.

2. The method according to claim 1, comprising administering to the subject at least 1 consecutive course of the interferon-based therapeutic agent.

3. The method according to claim 1, comprising intermittently administering to the subject a plurality of consecutive courses of the interferon-based therapeutic agent.

4. The method according to any one of claims 1-3, wherein the subject has been diagnosed as having cancer, but has undergone an anti-cancer treatment and is in remission.

5. The method according to claim 4, wherein the anticancer treatment is surgery, chemotherapy, radiation therapy, immunotherapy, or a combination thereof.

6. The method according to claim 4, the remission is a complete remission, e.g., no visible lesion (tumor) is detected in the subject.

7. The method according to any one of claims 1-6, wherein the interferon-based therapeutic agent comprises an interferon or a mutant or derivative thereof, or comprises a nucleic acid molecule encoding an interferon or a mutant or a derivative thereof, or comprises a substance promoting the generation of an endogenous interferon.

8. The method according to any one of claims 1-7, wherein the interferon is a Type I, Type II or Type III interferon, such as interferon α, interferon β, interferon γ or interferon λ, preferably interferon α.

9. The method according to any one of claims 1 to 8, wherein the interferon-based therapeutic agent comprises interferon α 2a, interferon α 2b, interferon α 1b, interferon λ, or a mutant or derivative thereof.

10. The method according to any one of claims 1 to 9, wherein the interferon or the mutant or derivative thereof is PEGylated.

11. The method according to any one of claims 1 to 10, wherein the interferon-based therapeutic agent is selected from the group consisting of P1101, Pegberon, Pegasys, Pegintron, Infergen, Novaferon, INTRONA, Roferon-A, Hapgen, PEGINFER and Peginterferon λ.

12. The method according to any one of claims 1 to 11, wherein the interferon-based therapeutic agent comprises an agonist of the TLRs, RLRs, and STINGs signaling pathways.

13. The method according to claim 12, wherein the interferon-based therapeutic agent is selected from the group consisting of GS-9620, GS-9688, RO7020531, RO6864018, TQ-A3334, JNJ-4964, SB9200, MIW815, DMXAA, MK-1454, and diABZI.

14. The method according to any one of claims 1-13, wherein in the consecutive treatment course, the interferon-based therapeutic agent is administered such that during substantially the entire course, the concentration of neopterin in the subject is higher than the concentration of neopterin before the first administration, for example approximately 110%, approximately 120%, approximately 130%, approximately 140%, approximately 150%, approximately 200%, approximately 250% or higher of the neopterin concentration before the first administration.

15. The method according to any one of claims 1-14, wherein the duration of the consecutive treatment course is the time period from the first administration to the last administration, plus about 5 in vivo half-lives of the therapeutic agent.

16. The method according to any one of claims 1-15, wherein the duration of the consecutive treatment courses is from about 1 week to about 24 weeks, preferably from about 1 week to about 12 weeks, further preferably from about 1 week to about 8 weeks, and yet further preferably about 2 weeks to about 6 weeks.

17. The method according to any one of claims 1-16, wherein the interval between the consecutive treatment courses is from about 1 week to about 24 weeks, preferably from about 1 week to about 12 weeks, further preferably from about 1 week to about 8 weeks, and yet further preferably about 2 weeks to about 6 weeks.

18. The method according to any one of claims 1-17, wherein the duration of each of the consecutive treatment courses is about 1 week to about 8 weeks, and the interval between the consecutive treatment courses is about 1 week to about 8 weeks.

19. The method according to any one of claims 1-18, wherein the duration of each of the consecutive treatment courses is about 2 weeks to about 6 weeks, and the interval between the consecutive treatment courses is about 2 week to about 6 weeks.

20. The method according to any one of claims 1-19, wherein the interferon-based therapeutic agent is administered for 2-25 or more consecutive treatment courses.

21. The method according to any one of claims 1-20, wherein the durations of the plurality of consecutive treatment courses are substantially the same.

22. The method according to any one of claims 1-21, wherein the intervals between the consecutive treatment courses are substantially the same.

23. The method according to any one of claims 1-22, wherein the additional anticancer agent is preferably for preventing cancer recurrence in the subject.

24. The method according to any one of any one of claims 1-23, wherein the additional anticancer agent is
i) a chemotherapeutic agent, such as an alkylating agent an alkylating agent: Nimustine, Carmustine, Lomustine, Cyclophosphamide, Ifosfamide, glyciphosphoramide, semustine; an antimetabolite: deoxyfluoguanosine, doxifluguanidine, 5-fluorouracil, mercaptopurine, thioguanine, cytarabine, fluguanosine, tegafur, Gemcitabine, carmofur, hydroxyurea, methotrexate, UFT, Ancitabine, capecitabine; an anti-tumor antibiotic: actinomycin D, doxorubicin, daunorubicin, Epirubicin, mitomycin, pelomycin, pingyangmycin, pirarubicin; a chemotherapeutic anti-tumor animal and plant ingredient: irinotecan, harringtonine, hydroxycamptothecin, Vinorelbine, paclitaxel, albumin paclitaxel, taxotere, topotecan, vincristine, vindesine, Vindesine, vinblastine, teniposide, etoposide, elemene; such as anti-tumor drug hormones: Atamestane, Anastrozole, Aminoglutethimide, Letrozole, Formestane, Metasterone, Tamoxifen; a chemotherapeutic miscellaneous agent: asparaginase, carboplatin, cisplatin, Dacarbazine, Oxaliplatin, Loxadine, Eloxatin, Mitoxantrone, or Procarbazine; or
ii) an immune checkpoint inhibitor such as an inhibitor of PD-1, PD-L1, CTLA4, for example, an antibody selected from: Nivolumab, Pembrolizumab, Atezolizumab, Durvalumab, Avelumab; or
iii) a small molecule targeting drug, such as imatinib, gefitinib, bortezomib, erlotinib, sorafenib, lenalidomide, Sunitinib, dasatinib, nilotinib, lapatinib, pazopanib, everolimus, vandetanib, crizotinib, verofinib, ruxolitinib, axitinib, vismodegib, carfilzomib, regorafenib, bosutinib, tofacitinib, carbotinib, panatinib, pomalidomide, trametinib, dabrafenib, Afatinib, Icotinib, Ibrutinib, Ceritinib, Idelaris, Apatinib, Pabuccilib, Levatinib, Axitinib, Icotinib , Apatinib, sonidegib, cobimetinib, osimertinib, alectinib, ixazomib; or
iv) a tumor-associated antigen-specific antibody such as Rituxan, Herceptin;
preferably, the anticancer agent is selected from oxaliplatin, epirubicin, paclitaxel and gemcitabine, and more preferably is gemcitabine.

25. The method according to any one of any one of claims 1-24, wherein the cancer is selected from leukemia (such as acute lymphocytic leukemia (ALL), acute myeloid leukemia (AML), chronic myeloid leukemia (CML), chronic lymphocytic leukemia, polycapillary leukemia), liver cancer, lung cancer, colorectal cancer, skin cancer, stomach cancer, breast cancer, prostate cancer, non-Hodgkin's lymphoma, melanoma, multiple myeloma, laryngeal papilloma, follicular lymphoma, AIDS-related Kaposi's sarcoma and renal cell carcinoma, preferably liver cancer, lung cancer, breast cancer, colorectal cancer or melanoma.

26. A pharmaceutical combination for use in preventing cancer recurrence in a subject, comprising an interferon-based therapeutic agent and an additional anticancer agent.

27. The pharmaceutical combination according to claim 26, which is for use in preventing cancer recurrence in a subject by the method according to any one of claims 1-25.

28. The pharmaceutical combination according to claim 26 or 27, wherein the interferon-based therapeutic agent comprises an interferon or a mutant or derivative thereof, or comprises a nucleic acid molecule encoding an interferon or a mutant or a derivative thereof, or comprises a substance promoting the generation of an endogenous interferon.

29. The pharmaceutical combination according to any one of claims 26-28, wherein the interferon is a Type I, Type II or Type III interferon, such as interferon α, interferon β, interferon γ or interferon λ, preferably interferon α.

30. The pharmaceutical combination according to any one of claims 26 to 29, wherein the interferon-based therapeutic agent comprises interferon α 2a, interferon α 2b, interferon α 1b, interferon λ, or a mutant or derivative thereof.

31. The pharmaceutical combination according to any one of claims 26 to 30, wherein the interferon or the mutant or derivative thereof is PEGylated.

32. The pharmaceutical combination according to any one of claims 26 to 31, wherein the interferon-based therapeutic agent is selected from the group consisting of P1101, Pegberon, Pegasys, Pegintron, Infergen, Novaferon, INTRONA, Roferon-A, Hapgen, PEGINFER and Peginterferon λ.

33. The pharmaceutical combination according to any one of claims 26 to 32, wherein the interferon-based therapeutic agent comprises an agonist of the TLRs, RLRs, and STINGs signaling pathways.

34. The pharmaceutical combination according to claim 33, wherein the interferon-based therapeutic agent is selected from the group consisting of GS-9620, GS-9688, RO7020531, RO6864018, TQ-A3334, JNJ-4964, SB9200, MIW815, DMXAA, MK-1454, and diABZI.

35. The pharmaceutical combination according to any one of claims 26-34, wherein the anticancer agent is
i) a chemotherapeutic agent, such as an alkylating agent an alkylating agent: Nimustine, Carmustine, Lomustine, Cyclophosphamide, Ifosfamide, glyciphosphoramide, semustine; an antimetabolite: deoxyfluoguanosine, doxifluguanidine, 5-fluorouracil, mercaptopurine, thioguanine, cytarabine, fluguanosine, tegafur, Gemcitabine, carmofur, hydroxyurea, methotrexate, UFT, Ancitabine, capecitabine; an anti-tumor antibiotic: actinomycin D, doxorubicin, daunorubicin, Epirubicin, mitomycin, pelomycin, pingyangmycin, pirarubicin; a chemotherapeutic anti-tumor animal and plant ingredient: irinotecan, harringtonine, hydroxycamptothecin, Vinorelbine, paclitaxel, albumin paclitaxel, taxotere, topotecan, vincristine, vindesine, Vindesine, vinblastine, teniposide, etoposide, elemene; such as anti-tumor drug hormones: Atamestane, Anastrozole, Aminoglutethimide, Letrozole, Formestane, Metasterone, Tamoxifen; a chemotherapeutic miscellaneous agent: asparaginase, carboplatin, cisplatin, Dacarbazine, Oxaliplatin, Loxadine, Eloxatin, Mitoxantrone, or Procarbazine; or
ii) an immune checkpoint inhibitor such as an inhibitor of PD-1, PD-L1, CTLA4, for example, an antibody selected from: Nivolumab, Pembrolizumab, Atezolizumab, Durvalumab, Avelumab; or
iii) a small molecule targeting drug, such as imatinib, gefitinib, bortezomib, erlotinib, sorafenib, lenalidomide, Sunitinib, dasatinib, nilotinib, lapatinib, pazopanib, everolimus, vandetanib, crizotinib, verofinib, ruxolitinib, axitinib, vismodegib, carfilzomib, regorafenib, bosutinib, tofacitinib, carbotinib, panatinib, pomalidomide, trametinib, dabrafenib, Afatinib, Icotinib, Ibrutinib, Ceritinib, Idelaris, Apatinib, Pabuccilib, Levatinib, Axitinib, Icotinib , Apatinib, sonidegib, cobimetinib, osimertinib, alectinib, ixazomib; or
iv) a tumor-associated antigen-specific antibody such as Rituxan, Herceptin;
preferably, the anticancer agent is selected from oxaliplatin, epirubicin, paclitaxel and gemcitabine, and more preferably is gemcitabine.
